# EUROPEAN PATENT APPLICATION

(11) **EP 2 098 865 A1**
(43) Date of publication of application: **09.09.2009**
(21) Application number: 09380040.7
(22) Date of filing: 04.03.2009
(51) Int. Cl.: G01N 33/569, C07K 14/195, C07K 16/12

(54) **Procedure for the detection of legionella spp using polyclonal antibodies**

(30) Priority: 04.03.2008 ES 200800628
(71) Applicant: Guserbiot S.L.U., 01015 Vitoria Alava (ES)
(72) Inventor: Barbero Mangas, Francisca, 01015 Vitoria Alava (ES); Izaguirre Goyoaga, Jon Kepa, 01015 Vitoria Alava (ES); Llosa Cruz, Armando, 01015 Vitoria Alava (ES)
(74) Representative: Carvajal y Urquijo, Isabel

(57) **Abstract**

The present invention provides an antigen which binds to an anti-*Legionella* spp polyclonal antibody, a procedure for obtaining the antigen, the use of said antigen as an analyte for the detection of *Legionella* spp and a procedure for the detection of *Legionella* spp in a biological sample based on the use of an polyclonal antibody, which enables to perform a detection at genus level.

## Description

### FIELD OF THE INVENTION

The present invention refers to the field of water analysis, and more specifically to the detection of Legionella.

### BACKGROUND OF THE INVENTION

Legionella is the etiological agent of the Legionnaires' disease, a human infection similar to the pneumonia which was first characterized in 1976 and which has been recognized, since then, as an important pulmonary pathogen responsible for a large number of nosocomial infections.

Within the Legionellae there are several species, being *Legionella pneumophila* the most important of them because it is the one that produces most of the cases of pneumonia in human beings. However, there exist some other 30 species of *Legionella* with pathogenic potential and among them there are several serogroups, which in the case of *Legionella pneumophila* more than 14 serogroups have been identified.

*L. pneumophila* is a gram-negative bacterium which can be usually found in aquatic environments, especially in those environments close to human activities such as cooling towers, water distribution systems, and underground water.

Nowadays, various techniques have been developed for the detection of Legionella such as bacterial cultures; however, given that these bacteria need some special requirements for its development, making it very difficult to isolate these bacteria in the commonly used microbiological medium, most laboratories do not use these cultures or do so inadequately (Feeley JC. Charcoal-yeast extract agar: primary isolation medium for Legionella pneumophila. J Clin Microbiol 1979; 10:437-441).

Recently, molecular detection techniques based on PCR are being used, particularly based on RT-PCR which detects legionella through the amplification of different genetic targets (Nele Wellinghausen, et al. "Detection of legionellae in Hospital water samples by quantitative Real-Time Light Cycler PCR. Applied and Environmental Microbiology, VOL. 67, No. 9, sep. 2001, p3985-3993). By using these techniques, the results are easily obtained in the laboratory; however, most of targets used are usually quiescent when the microorganism is in its environment, thus not being useful for the diagnosis of legionella in environmental samples.

### DESCRIPTION OF THE INVENTION

The present invention provides an antigen that is engaged with an anti-Legionella spp antibody, a procedure for obtaining the antigen and the use of said antigen as an analyte for the detection of *Legionella* spp. The present invention offers a procedure for the detection of *Legionella* spp, based on the use of a polyclonal antibody, which allows a fast (15-30 minutes) and highly sensitive detection at genus level. The system enables to detect *Legionella* spp either in suspension or recovered from a 0.45 µm filter, once the sample has been filtered in a filtration tower. In this way, it is possible to reach a 10⁷ UFC/l sensibility, from problem samples with a minimal concentration of 10⁴ UFC/ml and a sample volume of 1000 ml. Therefore, it is a useful device for the confirmation of the presence of *Legionella* in grown cultures or colonies in the ordinary control procedure of these microorganisms in laboratories.

Accordingly, in a first aspect, the present invention relates to an antigen which binds to an anti-*Legionella* spp antibody, where said antigen (antigen of the present invention) comprises the ribosomal protein S3 of the subunit 30S, ribosomal protein S4 of the subunit 30S and the elongation factor Tu.

In a second aspect, the present invention refers to a procedure for obtaining an antigen which binds to an anti-*Legionella* spp. antibody (antigen of the present invention), **characterized in that** said antigen is obtained by cell lysis from a *Legionella pneumophila* culture.

In a more specific aspect of the present invention, cell lysis is realized with detergents, lytic enzymes, antibiotics, heat and/or sonication.

In a more specific aspect, detergents used in the present invention are selected among SDS, tween, Triton X-100, sodium deoxicolate and/or nonidet.

In a more specific aspect, lytic enzymes used in the present invention are selected among lysozyme, proteinase k and/or mutanolysin.

In a more specific aspect, antibiotics used in the present invention are selected among polymyxin, colistin and/or surfactin.

In a third aspect, the present invention relates to the use of an antigen obtained using the procedure described in the present invention as an analyte for the detection of *Legionella* spp.

In a fourth aspect, the present invention relates to a procedure for the detection of *Legionella* spp in a biological sample by using an *anti-Legionella* polyclonal antigen **characterized in that** it comprises the following stages:
a) previous treatment of the biological sample
b) contacting the biological sample treated in a) with an *anti-Legionella* antibody
c) detecting the antigen-antibody compound

In a more specific aspect of the present invention, the anti-*Legionella* polyclonal antibody reacts with the ribosomal protein antigens S3 of the subunit 30S, ribosomal protein antigens S4 of the subunit 30S and the elongation factor Tu.

In a more specific aspect of the present invention, the *anti-Legionella* antibody is conjugated to colloidal gold particles.

In a more specific aspect of the present invention, the anti-*Legionella* polyclonal antibody according to any of the claims 8-10, wherein the *anti-Legionella* antibody is immobilized acting as a capture antibody in an immunochemical system.

In a more specific aspect of the present invention, step a) of previous treatment of the biological sample consists of a cell lysis.

In a more specific aspect of the present invention, cell lysis of step a) is produced with detergents, lytic enzymes, antibiotics, heat and/or sonication.

In a more specific aspect, detergents used in the present invention are selected among SDS, tween, Triton X-100, sodium deoxicolate and/or nonidet.

In a more specific aspect, lytic enzymes used in the present invention are selected among lysozyme, proteinase k and/or mutanolysin.

In a more specific aspect, antibiotics used in the present invention are selected among polymyxin, colistin and/or surfactin.

In a more specific aspect, the biological sample of the present invention is a water sample.

In a more specific aspect, the biological sample of the present invention is in liquid suspension.

In a more specific aspect, the biological sample of the present invention is in a filter.

### DESCRIPTION OF THE DRAWINGS

Figure 1 shows the titration of polyclonal serum against intact cells.
Figure 2 shows the titration of polyclonal serum against lysed cells.
Figure 3 relates to the titration of polyclonal serum against the lysed cells. Cross-reactivity.
Figure 4 shows the characterization of the antibody obtained by western blotting. MW: molecular weight markers, A: Western Blot with α-*Legionella serum;* 1: Raw extract of *Escherichia coli,* 2: Raw extract of *Escherichia coli,* 3: Extract of *Legionella pneumophila* treated with TCA-acetone, 4: Raw extract of *Legionella pneumophila,* B: 12.5 % polyacrylamide gel. 1: Raw extract of *Escherichia coli,* 2: Raw extract of *Campylobacter coli,* 3: Extract of *Legionella pneumophila* treated with TCA-acetone, 4: Raw extract of *Legionella pneumophila.*
Figure 5 shows the detection limit assay (10⁷ UFC/l)

### DETAILED DESCRIPTION OF THE INVENTION

The present invention provides an antigen which binds to an anti-*Legionella* spp antibody, a procedure for the production of the antigen and the use of said antigen as an analyte for the detection of *Legionella* spp. The present invention provides a procedure for the detection of *Legionella* spp by using a polyclonal antibody in water samples by means of an immunochromatographic system and/or ELISA.

The present examples are provided as illustrative and non-limiting examples of the invention.

### Example 1: antigen preparation

The origin of the antigen was a culture of 48 hours at 37°C and 5% of CO₂ in BCYE agar from a streak of *Legionella pneumophila* ATCC 33152. After that period, each plate was resuspended in 1 ml of Ringer's solution until a suspension of about 10⁹ UFC/ml was obtained. The suspension was washed three times with Ringer's solution by centrifugation at 14000 g and recovery of cell pellet so as to eliminate the components typical to the culture medium.

The antigen was obtained by cell lysis with detergent (sodium dodecyl sulfate-SDS). To that end, the cell pellet resulting from the 1ml of suspension and washing was resuspended in 1ml of 1% SDS solution and left to act for 10 minutes at room temperature. After that time, it was filtered using a 0.22 µm pore filter to eliminate the unlysed cells.

The filtering suspension was characterized as regards protein concentration using the EZQ method (Molecular Probes) and denaturing polyacrylamide gel electrophoresis (SDS-PAGE) followed by staining with Sypro Ruby.

### Example 2: Obtaining the antibody

For obtaining the antibody, first, the rabbit was immunized, to that end 2.5 kg New Zealand White female rabbits (*Orthyctolagus cuniculus*) were administered different doses according to the pattern described in table 1.

**Table 1: Rabbit immunization pattern**

| | Day | Amount of immunized protein (mg) |
|---|---|---|
| Preimmune bleeding | 0 | - |
| 1^{st} immunization | 0 | 0.8 |
| 2^{nd} immunization | 14 | 0.5 |
| 1^{st} bleeding | 24 | - |
| 3^{rd} immunization | 44 | 0.5 |
| 2^{nd} bleeding | 54 | - |
| 4^{th} immunization | 74 | 0.5 |
| Final exsanguination | 84 | - |

Day 0: Preimmune bleeding
   5-7 ml blood extraction from the auricular artery. No anesthesia application.
Day 0: 1^{st} subcutaneous immunization
   0.8 mg of protein + Freund's complete adjuvant
   No anesthesia application.
Day 14: 2^{nd} subcutaneous immunization
   0.5 mg of protein + Freund's incomplete adjuvant
   No anaesthesia needed.
Day 24: 1^{st} bleeding
   5-7 ml blood extraction from the auricular artery.
   No anesthesia needed.
Day 35: 3^{rd} subcutaneous immunization
   0.5 mg of protein + Freund's incomplete adjuvant
   No anesthesia needed.
Day 45: 2^{nd} bleeding
   5-7 ml blood extraction from the auricular artery.
   No anesthesia needed.
Day 56: 4^{th} subcutaneous immunization
   0.5 mg of protein + Freund's incomplete adjuvant
   No anesthesia needed.
Day 66: Final exsanguination

Cardiac exsanguination Approximately 150 ml of blood were obtained. Preanesthesia with intramuscular Dormicun (Midazolam) was applied in the gluteus. Once the animal was relaxed, Imalgène 1000 (ketamine) i.m. anesthesia was applied in the gluteus.
Once the blood was obtained, the serum was obtained by coagulation. The immunoglobulins were purified by means of protein A resin. The purification was made at half scale (10ml) using the Montage Prosep Kit (Millipore) or at a small scale 1ml in Protein A column using the ÄKTA Explorer chromatographic system (GE healthcare).

The antibody was eluted in 0.3 M pH 3 citrate buffer neutralized with tris 1 M. A buffer change was made by means of ultrafiltration with a 60 kDa pore size (Millipore) tangential membrane device.

### Example 3: Antibody characterization

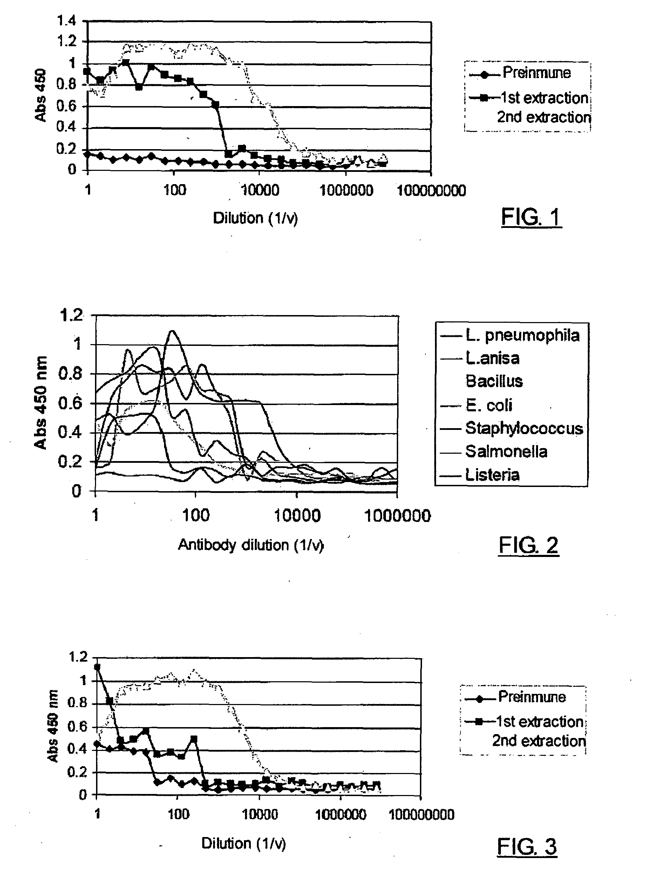

Both the serum and the purified antibody were characterized using direct ELISA. The characterization of the antiserum produced for the development is shown in figures 1 and 2.

The titers (the serum maximum dilution at which differential signal is presented against preimmune serum) of the production are shown in table 2.

**Table 2: titers**

| Antigen | 1^{st} Extraction | 2^{nd} Extraction |
|---|---|---|
| Unlysed *Legionella* | 256 | 16000 |
| Lysed *Legionella* | 4000 | 256000 |

The antibody was characterized using ELISA assay for assessing the maximum reactivity against different microorganisms extracts prepared in a similar way. Results are shown in Figure 3.

### The following strains were assessed:

*Legionella pneumophila* ATCC 33152, *Legionella anisa* ATCC 35292, *Bacillus subtilis* CECT 498, *Escherichia coli* CECT 10536, *Staphylococcus aureus* CECT 828, *Listeria monocytogenes* CECT 911.

Relative titers of each one of the assessed species are shown in table 3:

**Table 3**

| Species | Titer |
|---|---|
| *Legionella pneumophila* ATCC 33152 | 8000 |
| *Legionella anisa* ATCC 35292 | 1000 |
| *Bacillus subtilis* CECT 498 | 128 |
| *Escherichia coli* CECT 10536 | 500 |
| *Staphylococcus aureus* CECT 828 | 16 |
| *Salmonella typhimurium* CECT 443 | 128 |
| *Listeria monocytogenes* CECT 911 | <1 |

Higher reactivity was observed in assessed Legionella species, thus verifying that there exists a dilution 1:1000 serum 1µg/ml of antibody where the antibody specifically belongs to the *Legionella* type represented by the species assessed.

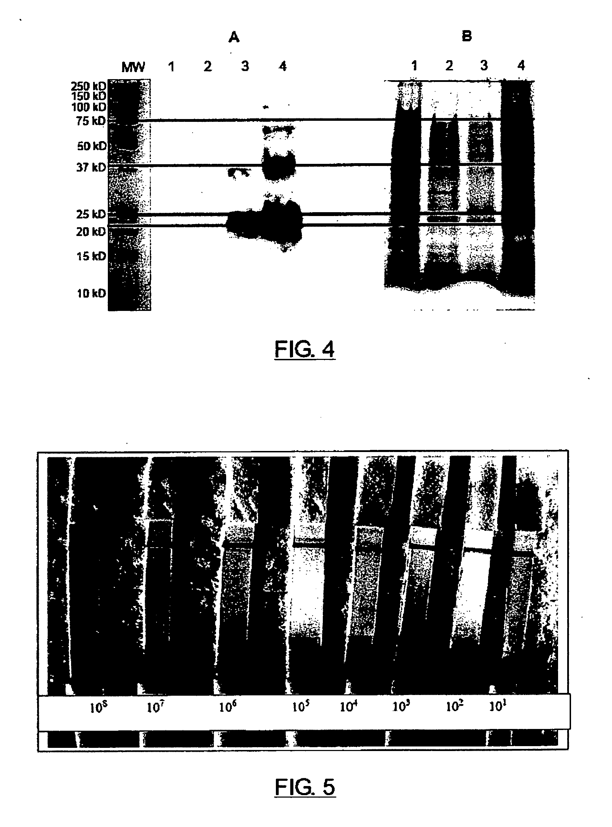

For assessing both the cross-reactivity and the proteic components acting as antigens in the detection, it was performed an analysis by Western Blotting of the antibody obtained (figure 4), thus verifying the antibody lack of cross-reactivity against *Escherichia coli* and *Campylobacter coli.* Three main reactive components were observed.

In order to identify the reactive antigens, the protein bands of the electrophoresis gel compatible with the reactivity in Western Blotting were pricked. The components identified by MALDI-TOF are shown in table 4:

**Table 4**

| Molecular weight (Da) | Identification | Species |
|---|---|---|
| 24159 | 30S ribosomal protein S3 | *Legionella pneumophila* |
| 23474 | 30S ribosomal protein S4 | *Legionella pneumophila* |
| 43344 | Elongation factor Tu | *Legionella pneumophila* |

### Example 4: Detection of Legionella by means of an immunochromatographic device.

The assess sample was a cell suspension from a colony grown on plates or on a 0.22 µm filter used in the filtration of 1000ml of water. The sample underwent one of the following lysis treatment procedures:
Heating at 100°C 10 minutes
Treatment with 5.3 mg/ml polymyxin + NaCl 9 g/l + heating at 100 °C 10 minutes.

The device developed was made under the following protocol:
A) Application of the antibodies to the membrane
   - Cutting of the roll in 20 cm strips with a right angle cut. HiFlow (Millipore SHF2400225) membranes were used.
   - Dilution of the antibodies in a 10 mM, pH 7.4 phosphate buffer according to the following proportion:
      - Control line: Polyclonal Goat anti-Rabbit (Dakocytomation Z0421) direct dilution. For 500 strips it was necessary a 500 µl. volume from a 125 µl volume of antibody. The volume for each 30 cm strip was 30 µl (60 strips)
      - Capture line: Polyclonal anti *Legionella* spp. (own development). It was necessary a 500 µl volume in 30 µl lots. Working dilution 1:1.
   - Application of a control line and a capture line of antibodies on the membrane at the necessary concentration with the Biodot (Volume rate/cm: 1 µl/cm).
   - Drying of the membranes for 1 hour at room temperature.
   - Introduction of the membrane for 30 minutes in a 0.5% Triton X 100 solution.
   - Rinsing of the membrane in distilled water and drying for 30 minutes at 30° C.
B) Conjugation of the antibody to gold and preparation of the conjugate pad.
   - Preparation of a tube with 15 ml of the desired volume (150 µl/strip) of colloidal gold (pH 9) (Colloidal Gold 40 nm. Schleicher & Schuell Ref. 10534251) and addition of 1.5 ml of the antibody dilution of our own development drop by drop according to the proportion calculated in the titration. Dilution of the antibody in Na-Borate 2mM pH 9.
   - Agitation for 10 minutes.
   - Addition of BSA of a 10% stock solution to reach a 1% final concentration.
   - Mild agitation for another 10 minutes.
   - Conjugate centrifugation at 16000 x g for 45 minutes at 4°C.
   - Pellet resuspension in TBS with 1% BSA concentrating 5 times the original volume.
   - Recutting of the conjugate membrane (Millipore Glass Fiber Conjugate, 10 x 300 mm GFCP103000) in 30 cm portions.
   - Application of 25 µl of the mix to each strip placed on a glass surface every 0.5 cm Or automatic application using the same parameters.
   - Drying at 40°C for 2 hours.
C) Membrane mounting
   The commercial pad 17 x 300 mm Absorbent Pad CFSP173000 was used as absorbent pad and the commercial pad 17x 300 mm Sample Pad CFSP173000 was used as sample pad. The strips were mounted according to specifications in the following order:
   - Peel off the adhesive protector at the top of the membrane.
   - Stick the absorbent strip, ensuring that it overlaps the nitrocellulose layer.
   - Peel off the protector of the part corresponding to the conjugate and the absorbent.
   - Stick the conjugate strip (previously dried).'
   - Stick the absorbent strip.
   - Place the laminate.
   - Cut the ensemble in 0.5 cm units.

### Example 5: Detection assay in real samples

A total of 15 samples were analyzed. Said samples underwent a lysis treatment and were inoculated at three contamination levels. No inhibition of the immunochemical reaction was observed in any of the samples, so it was deduced that at least in the analyzed samples there were no inhibitors preventing the assay. Results are shown in table 5.

**Table 5: X 100 Filtered and concentrated. L Lysed, UL: Unlysed. UD: Undetermined**

| SAMPLE | X100 | 0 UFC/L | | 100 UFC/L | | 1000 UFC/L | | 10⁶ UFC/L | |
|---|---|---|---|---|---|---|---|---|---|
| | | L | UL | L | UL | L | UL | L | UL |
| 162-228 | - | + - | + - | - | - | - | - | - | - |
| 162-229 | - | + - | + | - | - | - | - | - | - |
| 162-227 | - | + - | + - | - | - | - | - | - | - |
| 162-273 | - | + - | - | - | - | - | - | - | - |
| 199-52 | - | - | - | - | - | - | - | - | - |
| 199-53 | - | - | - | - | - | - | - | - | - |
| 199-54 | - | - | - | - | - | - | - | - | - |
| 89 | UD | | | | | | | | |
| | - | - | - | - | - | - | - | - | |
| 192-1 | UD | - | - | - | - | - | - | - | - |
| 192-2 | UD | - | - | - | - | - | - | - | - |
| 199 | UD | - | - | - | - | - | - | - | - |
| 251 | UD | - | - | - | - | - | - | - | - |
| UV-M | UD | - | - | - | - | - | - | - | - |

The calibration of the band allotment had the following results. According to this assay the detection limit is at 10⁷ UFC/ml (figure 5).

Due to the low sensitivity of the device different simple or combined lysis procedures were applied to different dilutions of *Legionella*. Suspensions were filtered through 0.22 µm to detect the unlysed fraction and were immobilized in a polystyrene plate. The assay consisted in a serial dilution of the serum corresponding to the antibody of our own development according to example 2, the comparative assessment of each antigen in relation to a control serum. The result of these assays was the determination of a minimal serum dilution (titer) at which a specific *Legionella* concentration was detected. Therefore, the higher this value, the higher the serum reactivity against an antigen because reactivity was kept at a high dilution. 50% serial dilutions ranging from 40 to 20000 were made. To that end, the titer for each concentration and lysis procedure ranged from >20000 (maximum reactivity) to <40 (null or very low reactivity).

For the titer calculation, there were considered only those dilutions where the problem serum was higher than the control in at least 50% in absorbance units.

The treatments applied were the following:
- Treatment A: 1% SDS. Treatment for 10 minutes. Compatible with a field device.
- Treatment B: 1% Tween 20. Treatment for 10 minutes. Compatible with a field device.
- Treatment C: Lysis with Lisozyme. 30 minutes treatment at 37°C. Compatible with a field device.
- Treatment D: Lysis with Proteinase K 30 minutes treatment at 60°C. Compatible with a laboratory procedure.
- Treatment E: Heating at 95°C 10 minutes Compatible with a laboratory procedure.
- Treatment F: Sonication in an ultrasound bath 30 minutes at maximum power. Compatible with a laboratory procedure.

Additionally, the following combined treatments have also been tested

| | | | | | |
|---|---|---|---|---|---|
| A+EA+F | C+A | C+A+E | | C+A+F | |
| B+E | B+F | C+B | C+B+E | C+B+F | |
| C+E | C+F | D+A | D+A+E | D+A+F | |
| D+E | D+F | D+B | D+B+E | D+B+F | F+E |

Results are shown in table 6.

**Table 6**

| Table 6: Titer of the antibody against treated dilutions (UFC/ml) of the antigen | | | | | | | |
|---|---|---|---|---|---|---|---|
| | 10⁷ | 10⁶ | 10⁵ | 10⁴ | 10³ | 10² | 10 |
| A | >20000 | >20000 | 10000 | 10000 | <40 | <40 | <40 |
| A+E | >20000 | >20000 | >20000 | 10000 | 640 | 640 | 640 |
| A+F | >20000 | >20000 | >20000 | 5000 | 40 | 40 | <40 |
| B | <40 | <40 | <40 | <40 | <40 | <40 | <40 |
| B+E | >20000 | >20000 | 40 | 40 | 40 | 40 | 40 |
| B+F | 640 | 320 | 320 | 160 | 160 | 160 | 80 |
| C | >20000 | >20000 | >20000 | 2560 | 2560 | 2560 | 40 |
| C+A | 5000 | 5000 | 5000 | 2560 | 2560 | <40 | <40 |
| C+A+E | >20000 | 10000 | 10000 | 10000 | 2560 | 640 | 320 |
| C+A+F | >20000 | >20000 | >20000 | >20000 | 10000 | 40 | 40 |
| C+B | 5000 | >20000 | >20000 | >20000 | <40 | <40 | <40 |
| C+B+E | >20000 | >20000 | 2560 | >20000 | >20000 | 160 | 160 |
| C+B+F | >20000 | >20000 | >20000 | >20000 | >20000 | <40 | <40 |
| C+E | 2560 | 2560 | 2560 | 1280 | 640 | 640 | 640 |
| C+F | >20000 | >20000 | >20000 | >20000 | 10000 | <40 | <40 |
| D | >20000 | >20000 | <40 | <40 | <40 | <40 | <40 |
| D+A | >20000 | >20000 | 40 | 40 | 40 | 40 | 40 |
| D+A+E | >20000 | >20000 | 40 | <40 | <40 | <40 | <40 |
| D+A+F | >20000 | >20000 | 40 | <40 | <40 | <40 | <40 |
| D+B | >200C0 | 5000 | <40 | <40 | <40 | <40 | <40 |
| D+B+E | >20000 | 640 | 640 | <40 | <40 | <40 | <40 |
| D+B+F | >20000 | 80 | 80 | <40 | <40 | <40 | <40 |
| D+E | >20000 | >20000 | >20000 | 640 | 320 | 320 | 320 |
| D+F | >20000 | >20000 | 1280 | -1280 | 1280 | 1280 | <40 |
| E | >20000 | >20000 | >20000 | >20000 | 5000 | 2560 | 1280 |
| F | >20000 | >20000 | 2560 | 640 | 640 | 640 | 640 |
| E+F | >20000 | >20000 | >20000 | 2560 | 2560 | 2560 | 2560 |

From this assay it can be concluded that:
- The three higher assay concentrations (107 UFC/ml, 106 UFC/ml and 105 UFC/ml) can be detected using most of the applied treatments.
- The three low assay concentrations (104 UFC/ml, 103 UFC/ml y 102 UFC/ml) can be more easily detected using Lisozyme-Tween and heating combined treatements.
- The lower concentration (10 UFC/ml) can be more easily detected using physical treatments (heating and sonication) and using the SDS-heating combined treatment.

## Claims

1. Antigen which binds to an anti-*Legionella* spp antibody **characterized in that** said antigen comprises the ribosomal protein S3 of the subunit 30S, ribosomal protein S4 of the subunit 30S and the elongation factor Tu.

2. Procedure for obtaining an antigen which binds to an anti-*Legionella* spp. antibody according to claim 1, **characterized in that** said antigen is obtained by cell lysis from a *Legionella pneumophila* culture.

3. Procedure for obtaining an antigen which binds to an anti-*Legionella* spp. according to claim 2, wherein the cell lysis is performed with detergents, lytic enzymes, antibiotics, heat and/or sonication.

4. Procedure for obtaining an antigen which binds to an anti-*Legionella* spp. antibody according to claim 3, wherein the detergents are selected among SDS, tween, Triton X-100, sodium deoxicolate and/or nonidet.

5. Procedure for obtaining an antigen which binds to an anti-*Legionella* spp. antibody according to claim 3, wherein the lytic enzymes are selected among lysozyme, proteinase k and/or mutanolysin.

6. Procedure for obtaining an antigen which binds to an anti-*Legionella* spp. antibody according to claim 2, wherein the antibiotics are selected among polymyxin, colistin and/or surfactin.

7. Use of an antigen obtained by means of a procedure according to any of the claims 2-6 as analyte for the detection of *Legionella* spp.

8. Procedure for detecting *Legionella* spp. in a biological sample using an anti-*Legionella* polyclonal antibody **characterized in that** it comprises the following stages:
d) previous treatment of the biological sample
e) contacting the biological sample treated in a) with an anti-*Legionella* antibody
f) detecting the antigen-antibody compound

9. Procedure for detecting *Legionella* spp. in a biological sample using an anti-*Legionella* polyclonal antibody according to claim 8, wherein the *anti-Legionella* polyclonal antibody reacts with the ribosomal protein S3 antigens of the subunit 30S, and the ribosomal protein S4 of the subunit 30S and the elongation factor Tu.

10. Procedure for detecting *Legionella* spp. in a biological sample using an anti-*Legionella* polyclonal antibody according to any of the claims 8-9, wherein the anti-*Legionella* antibody is conjugated to colloidal gold particles.

11. Procedure for detecting *Legionella* spp. in a biological sample using an anti-*Legionella* polyclonal antibody according to any of the claims 8-10, wherein the anti-*Legionella* antibody is immobilized acting as capture antibody in an immunochemical system.

12. Procedure for detecting *Legionella* spp. in a biological sample using an anti-*Legionella* polyclonal antibody according to any of the claims 8-11, wherein the stage a) of previous treatment of the biological sample consists of cell lysis.

13. Procedure for detecting *Legionella* spp. in a biological sample using an anti-*Legionella* polyclonal antibody according to claim 12, wherein the cell lysis is performed with detergents, lytic enzymes, antibiotics, heat and/or sonication.

14. Procedure for detecting *Legionella* spp. in a biological sample using an anti-*Legionella* polyclonal antibody according to claim 13, wherein the detergents are selected among SDS, tween, Triton X-100, sodium deoxicolate and/or nonidet.

15. Procedure for detecting *Legionella* spp. in a biological sample using an anti-*Legionella* polyclonal antibody according to claim 13, wherein the lytic enzymes are selected among lysozyme, proteinase k and/or mutanolysin.

16. Procedure for detecting *Legionella* spp. in a biological sample using an anti-*Legionella* polyclonal antibody according to claim 13, wherein the antibiotics are selected among polymyxin, colistin and/or surfactin.

17. Procedure for detecting *Legionella* spp. in a biological sample using an anti-*Legionella* polyclonal antibody according to any of the claims 8-16, wherein the biological sample is a water sample.

18. Procedure for detecting *Legionella* spp. in a biological sample using an anti-*Legionella* polyclonal antibody according to claim 17, wherein the biological sample is in liquid suspension.

19. Procedure for detecting *Legionella* spp. in a biological sample using an anti-*Legionella* polyclonal antibody according to claim 17, wherein the sample is in a filter.
